Europäisches Patentamt

European Patent Office

Office européen des brevets

(19)

(11) Publication number: **0 432 871 A2**

# EUROPEAN PATENT APPLICATION

(12)

(21) Application number: 90307651.1

(22) Date of filing: **12.07.90**

(51) Int. Cl.5: **A61L 2/26**, C12Q 1/22

The title of the invention has been amended (Guidelines for Examination in the EPO, A-III, 7.3).

(30) Priority: **16.11.89 US 437545**

(43) Date of publication of application:
**19.06.91 Bulletin 91/25**

(84) Designated Contracting States:
**DE FR GB IT**

(71) Applicant: **PYMAH CORPORATION**

**89 Route 206**
**Somerville New Jersey 08876(US)**

(72) Inventor: **Hanafin, Paul**

**deceased(US)**

(74) Representative: **Coxon, Philip et al**
**Eric Potter & Clarkson St. Mary's Court St.**
**Mary's Gate**
**Nottingham NG1 1LE(GB)**

(54) **Indicator test pack for ethylene oxide sterilisation.**

(57) An easy open syringe for use in testing an ethylene oxide sterilizer comprises a vented hollow container having an opening for receiving a sterilization indicator therein. A plunger with a plug covers the opening of the container to hold the sterilization indicator in the container. The syringe and its enclosed indicator are positioned in a sterilizable peel pouch. After completion of a sterilization cycle, the container may be removed from the envelope and the plunger withdrawn from the container to remove the indicator from the easy open syringe for examination and determination of sterilization cycle efficiency.

Fig. 1

This invention relates to apparatus for receiving a sterilization indicator in an envelope. More particularly, this invention relates generally to sterilizer test packs. Specifically, this invention relates to a pseudo syringe which can be loaded with a sterilization indicator and then placed in a pouch or envelope wherein it is subjected to a sterilization cycle. After completion of the sterilization cycle, the present invention permits easy removal of the syringe from the envelope and the indicator from the syringe. this invention is particularly, though not exclusively, useful in the testing of an ethylene oxide (EO) sterilizer.

It is well known in the medical profession that many medical procedures require devices and instruments which are thoroughly sterilized before being used for their intended purpose. Not all such devices and instruments, however, can be sterilized in the same manner. Consequently, several different sterilization procedures are followed. The particular procedure of interest here sterilizes devices and instruments through exposure to ethylene oxide (EO) gas.

For EO sterilization, as with any sterilization procedure, the operator must be certain that sterilizing conditions are achieved. While absolute sterility (100% kill of all microbes) is theoretically unattainable because of the logarithmic nature of microbial death kinetics, minimum standards of sterilization have been recommended by cognizant organisations. For EO sterilization, specific procedural guidelines have been established and specially designed test packs have been suggested. Unfortunately, present procedures for using these test packs unnecessarily expose the operator to the toxic effects of ethylene oxide. Moreover, present procedures for testing EO sterilization cycle efficacy contemplate the use of biological indicators only. As discussed below, biological indicators possess certain disadvantages compared to other types of sterilization indicators, such as chemical integrators.

According to the Association for the Advancement of Medical Instrumentation (AAMI), an EO test pack should include a structure that simulates the items which are to be sterilized. Additionally, such a test pack should incorporate appropriate thermal and moisture barriers. With these considerations in mind, AAMI has recommended that an EO sterilizer test pack comprise a standard syringe into which a biological indicator has been placed. Further, AAMI recommends that the needle end of the syringe remain open and that the plunger be inserted into the syringe barrel in order to confine gas communication into the syringe through the opening at the needle end. The combination is then wrapped in a clean surgical towel and inserted into a peel pouch. The peel pouch and its contents are then subjected to an EO sterilization cycle. Subsequently, the biological indicator is examined to test the effectiveness of the sterilization cycle.

For the purposes of the AAMI test, any biological indicator well known in the art may be used. The AAMI procedure, however, does not contemplate the use of sterilization indicators other than biological indicators. For example, any test pack having a biological spore strip innoculated with an appropriate population (1,000,000 generally) of spores from the Bacillus subtilis organism may be used for the AAMI procedure. Although not specified as a sterilization sensor by AAMI, use of an integrator in a sterilization test pack has significant advantages over use of the more conventional, AAMI-specified biological indicator. Specifically, with an integrator, results of the sterilization equipment test may be obtained almost immediately after the test has been completed. In contrast, sterilization test results from a biological indicator may not be available for several days. One disadvantage of delays in obtaining test results is that hospitals do not typically possess a large enough inventory of equipment to permit shelving sterilized equipment for the length of time it takes to receive the sterilization test results. Thus, equipment which has undergone a faulty sterilization cycle could potentially be distributed for use before test results indicating cycle inadequacy could be obtained. Moreover, results from biological indicators are susceptible to greater statistical variations than are the relatively standardized, consistent results obtained from integrators. In addition to the above, integrators possess certain other advantages over both biological indicators and conventional chemical indicators. For example, crucial to EO cycle sterilization efficacy is the interdependence of the sterilization cycle variables of gas concentration, cycle time, temperature, and humidity. While many existing chemical indicators do provide test results relatively quickly, in the manner of integrators, they cannot be calibrated to provide a consistent indication of the efficacy of the sterilization equipment for all gas concentration-cycle time-temperature-humidity variations. Stated differently, while chemical indicators provide an accurate indication of sterilization cycle efficacy for some combinations of the variables set forth above, they do not provide an accurate indication of cycle efficacy for all combinations of the variables. In contrast, integrators, such as the Sterigage EO integrator manufactured by PyMaH Corporation, provide a quick, consistent and standardized indication of sterilization equipment efficacy for substantially all combinations of the four sterilization variables discussed above. Additionally, partial or intermediate indications can provide diagnostic information and can be used to assess the operational efficiency of the sterilization equipment itself.

Independent of the sterilization sensor being used, the object of any EO test pack is to make the contact of EO gas with the sterilization indicator or integrator being used as challenging as possible. It will be appreciated by the skilled artisan that this challenge is accomplished by using the barrier effects of several factors. Firstly, the test pack should provide thermal insulation for th sterilization sensor. The object here, of course, is to challenge the EO sterilization system's heating ability. Secondly, the test pack should provide diffusion control to ensure adequate penetration of the EO gas into the devices being sterilized. This is accomplished by requiring the EO to traverse a tortuous path before it contacts the biological indicator, or chemical integrator. Thirdly, the test pack should provide moisture control. Because moisture enhances the sterilization process, the removal of moisture from within the test pack provides a further challenge for the sterilizer. As is to be expected, all thse controls must cooperate and be functional in order for there to be an efficacious test pack. Typically, the test pack structure has been provided by using an otherwise operationally functional hypodermic syringe in which a biological indicator has been placed.

Once the sterilization cycle has been completed, the established procedure has been to remove the towel-wrapped syringe from the envelope, take the syringe out of the towel and disassemble the syringe. The biological indicator (in the AAMI procedure) is then removed from the syringe and incubated to test the efficacy of the sterilization cycle. This procedure, however, has several other disadvantages in addition to the disadvantages noted above. Foremost is operator exposure to EO gas. If the used test pack is opened shortly after the sterilization process is completed, rather than being properly aerated at elevated temperatures for approximately eight to twelve hours, the established procedure requiring removal and disassembly of the syringe can cause exposure of the operator to toxic EO gas for a significant amount of time. Ideally, however, the biological indicator or integrator should be removed from the test pack soon after sterilization without unwanted exposure to EO gas. Secondly, because the testpack is assembled from standard hypodermic syringes, a substantial portion of a hospital's supply of syringes may be used for the testing of sterilization equipment instead of for their intended purposes. This is wasteful.

US-A-4,739,881 recognised the need to provide a quick open syringe which could be disassembled while remaining in a sterilizable peel pouch envelope, and which could duplicate the accepted AAMI standards for EO sterilization while avoiding undue operator exposure to EO gas. However, the US-A-4,739,881 neither contemplated for nor provided for the use of an integrator, and therefore failed to incorporate the advantages noted above associated with integrators.

It is an object of this invention to provide an improved apparatus for receiving a sterilization indicator in an envelope.

According to one aspect of this invention there is provided an apparatus for receiving a sterilization indicator in an envelope, which apparatus comprises a container for receiving said indicator therein, said container being receivable in said envelope and being formed with an opening, and a plunger insertable into said container to cover said opening for holding said indicator in said container.

A preferred embodiment of the easy open syringe includes a vented hollow container into which a sterilization indicator is placed. The container may have an opening which removably receives a plunger that may be formed with a handle. The container with the sterilization indicator inside and the plunger inserted may be positioned inside a peel pouch envelope and the entire assembly may then be subjected to a sterilization cycle. Upon completion of the sterilization cycle, the syringe may be removed from the envelope by separating the sides of the envelope. The plunger in turn may then be withdrawn from the container to expose the sterilization indicator therein. Thus, the test unit contents may be quickly and easily removed to permit an early examination of the indicator and to thereby quickly assess sterilization cycle efficacy.

Thus, the preferred embodiment of the present invention recognises that there is a need to duplicate the AAMI test procedure in a way that allows for early retrieval of an integrator while reducing operator exposure to the toxic effects of ethylene oxide gas. Further, the present invention recognises a need for a test unit which will be relatively inexpensive and not require the destruction of syringes which could otherwise be used for their intended purposes.

Accordingly, it is an advantage of the present invention that it provides an easy open syringe which substantially satisfies current AAMI EO sterilization cycle test procedures. It is yet another advantage of the present invention that it provides an EO sterilizer test pack which provides for standardized sterilizer test results for various combinations of EO gas concentration, temperature, humidity, and cycle time within hours after completion of the sterilization cycle. Yet another advantage of the present invention is that it provides an easy open syringe for testing the efficacy of an EO sterilization cycle which is easily manufactured, cost-effective and which is easily used by the operator.

According to another aspect of this invention there is provided an easy opening apparatus for receiving a sterilization indicator which comprises:
a container for receiving said indicator therein, said container being formed with a vent and with an opening;
a plunger insertable into said opening for holding said indicator in said container; and
a resealable envelope to sealably hold said container therein.

According to another aspect of this invention there is provided an apparatus for receiving a sterilization indicator in an envelope which comprises:
a container formed with an opening for receiving said indicator therein, said container being receivable in said envelope;
a plunger insertable into said container to cover said opening to hold said indicator within said container, said plunger being retractable from said container to uncover said opening to release said indicator from said container; and
means for unsealing said envelope to release said container therefrom.

According to another aspect of this invention there is provided a method of testing the efficacy of a sterilizer comprising the steps of:
placing a sterilization indicator into an apparatus comprising a container formed with an opening and having a plunger covering said opening to hold said indicator within said indicator;
placing the combination of said indicator and said container into an envelope;
subjecting said envelope and its contents to a sterilization cycle of ethylene oxide;
removing said container from said envelope;
retracting said plunger from said container to uncover said opening for removing said indicator therefrom; and
observing said indicator to determine the effectiveness of the sterilizer.

Reference is now made to the accompanying drawings in which:-

Figure 1 is a perspective view of the easy open syringe placed with a peel pouch envelope;

Figure 2 is an exploded perspective view of the easy open syringe and its thermal insulation barrier;

Figure 3 is a side elevational view of the container and plunger combination with portions broken away for clarity;

Figure 4 is a top view of one embodiment of an integrator of the present invention; and

Figure 5 is an exploded view of the easy open syringe and peel pouch after the peel pouch envelope has been opened and the syringe has been manipulated for removal of the container's contents.

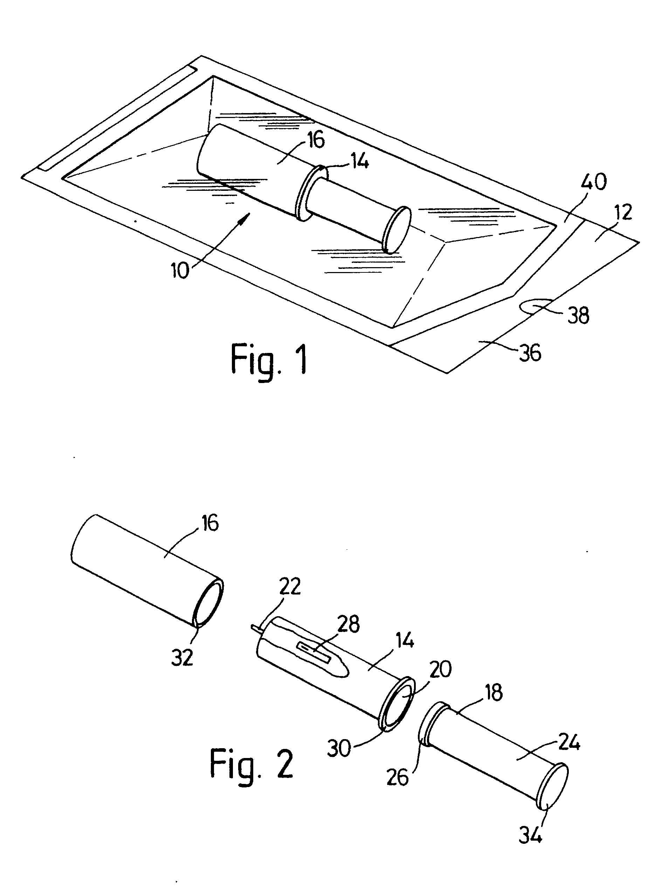

Referring initially to Figure 1, the easy open syringe of the present invention is shown generally designated 10. As seen in Figure 1, the easy open syringe 10 is enclosed within a peel pouch envelope 12.

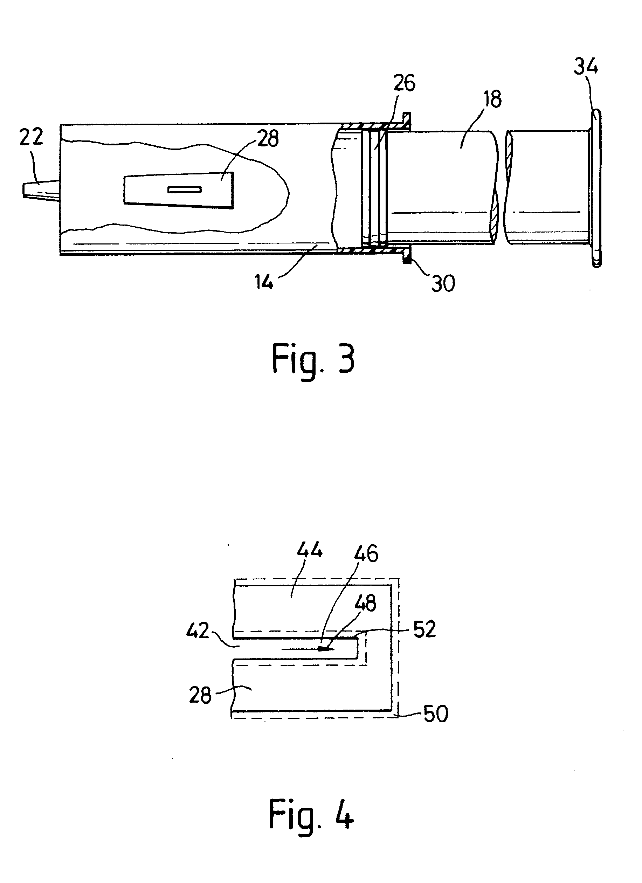

The major components of the easy open syringe 10 will be best appreciated by reference to Figure 2 in which an exploded perspective view of syringe 10 shows these components to be a container 14, a thermal insulator 16 and a plunger 18. Preferably, container 14 and plunger 18 are made of polypropylene. Alternately, however, they may be made from any of several polymers, e.g. nylon, polycarbonate or polysulfone. Thermal insulator 16, on the other hand, is preferably made of a paper material such as cardboard. Insulator 16 may, however, be made of a material commonly referred to as central supply room (CSR) wrap, foam plastic, cotton, sponge or cloth. Insulator 16 may also be formed as part of container 14 and still be within the intent of the present invention.

Figure 2 shows that container 14 is a hollow tubular structure. As can be seen from Figure 2, container 14 has an opening 20 which provides access to the hollow interior of container 14. Container collar 30 surrounds the periphery of container opening 20 and abuts edge 32 of insulator 16 when insulator 16 is positioned around container 14. The end of container 14 opposite opening 20 is closed except for a vent 22 which is intended to simulate the open needle end of a hypodermic syringe as required by the AAMI procedure. In order to regulate the diffusion control of syringe 10, vent 22 may be formed with varying apertures depending upon the particular needs and desires of the operator. Although vent 22, as shown in Figure 2, is formed to represent the needle end of a standard syringe, it will be appreciated by the skilled artisan that vent 22 may be a simple hole.

Plunger 18 of easy open syringe 10 is shown in Figure 2 with a handle 24 which is formed as an extension of the plunger 18. Plunger butt 34 extends radially from handle 24 and provides a handhold for plunger 18. Plunger 18 also comprises a plug 26 which is adapted to be inserted and held in the opening 20 of container 14 by an interference fit.

With plunger 18 removed, an integrator 28 can be inserted into the container 14 through opening 20. The skilled artisan will appreciate that any suitable biological or chemical indicator may be substituted for integrator 28 and still fall within the scope of the present invention. Plunger 18 can then be placed over opening 20 of container 14 to hold integrator 28 therein. As seen in Figure 4, integrator 28 comprises a base 44 with an aperture 42 formed therein. In the preferred embodiment, base 44 comprises aluminium foil. Disposed within base 44 is wick 46. Wick 46 may be attached to base 44 by any means well known in the art.

Preferably, wick 46 is glued to base 44. Wick 46 is impregnated with an integrating chemical which reacts with EO gas to change colour. The EO gas-wick 46 chemical reaction rate depends on predetermined levels of EO gas concentration/temperature/humidity conditions inside integrator 28. In the preferred embodiment, wick 46 comprises an impregnating chemical as disclosed in U.S. Patent No. 3,981,683. A suitable porous cover 50 is overlaid onto base 44 to cover base 44 and wick 46. Cover 50 is formed with window portion 52, which is disposed over wick 46 to permit visual inspection of wick 46.

The assembly of container 14 with plunger 18 is best seen with reference to Figure 3, while the entire assembly of syringe 10, including envelope 12, is best seen with cross reference to both Figures 1 and 3. In Figure 1, it will be seen that insulator 16 is positioned around container 14 to provide some degree of thermal protection for the contents of container 14. Various means, all well known in the pertinent art, may be used to position insulator 16 in surrounding association with the container 14. Preferably, as shown in Figure 2, insulator 16 is formed as a tubular member which can slide over the outside portion of container 14 until insulator edge 32 abuts collar 30 of container 14.

## OPERATION

In its operation, the easy open syringe 10 of the present invention allows for the placement of an integrator 28 inside container 14. Once integrator 28 has been placed within container 14, plunger 18 is inserted into the opening 20 of container 14 for the purpose of holding the integrator 28 inside container 14. A thermal insulator 16 is placed in surrounding relationship to container 14 for the purpose of providing thermal insulation for the integrator 28 which is being held within the container 14.

Once the easy open syringe 10 with its integrator 28 in place has been assembled, easy open syringe 10 is placed in peel pouch envelope 12 and the entire combination is subjected to an ethylene oxide sterilization cycle. Ethylene oxide (EO) gas enters container 14 through vent 22. As seen in Figure 4, EO gas inside container 14 enters integrator 28 through aperture 42 of base 44, to contact wick 46. When predetermined levels of EO gas concentration, temperature, and humidity conditions have been achieved, EO gas begins to react with the integrator chemical impregnated in wick 46. As this reaction progresses, wick 46 changes colour over time, the colour front starting substantially at aperture 42 and migrating, over time, in the direction of arrow 48. Cover 50 is scaled along window 52 to provide an indication of EO sterilization cycle efficacy based upon the extent of the colour front migration through wick 46 in the direction of arrow 48. It will be appreciated by the skilled artisan that if the wick 46 integrator chemical is properly selected, the rate of EO gas-wick 46 integrator chemical reaction will closely parallel the death rate of the bacteriological test spore of choice (Bacillus subtilis) over the same range of EO gas concentration-temperature-humidity conditions.

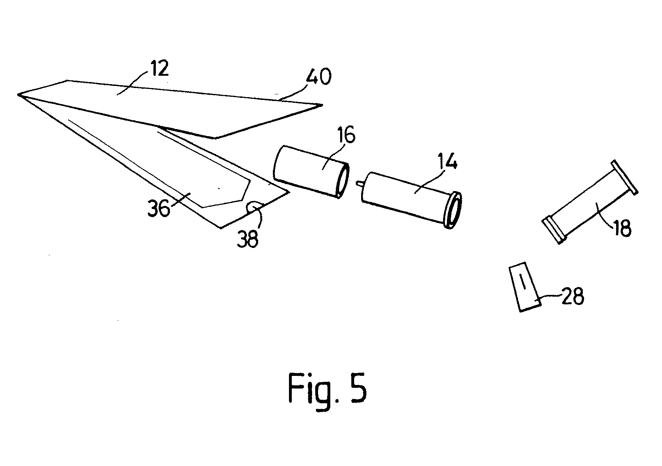

As shown in Figure 5, upon completion of sterilization cycle, the envelope 12 and the easy open syringe 10 contained therein are removed from the sterilizer, enabling the operator to grasp thumb notch 38 with one hand at bottom sheet 36 of envelope 12. With the other hand, the operator grasps top sheet 40 and separates top sheet 40 from bottom sheet 36, exposing syringe 10. Plunger 18 may now be slidably withdrawn from container 14, allowing for removal of integrator 28 for examination and assessment of sterilization cycle efficacy.

While the particular integrator test pack as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. An apparatus for receiving a sterilization indicator in an envelope, which apparatus comprises a container for receiving said indicator therein, said containing being receivable in said envelope and being formed with an opening, and plunger insertable into said container to cover said opening for holding said indicator in said container.

2. An apparatus according to Claim 1 comprising a handle extended from said plunger for withdrawing said plunger from said container to remove said indicator from said container.

3. An apparatus according to Claim 1 or 2 comprising an insulator mounted on a portion of said container to thermally insulate said container from its surroundings.

4. An apparatus according to any preceding claim wherein said plunger is formed with a plug for establishing an interference fit between said plunger and said container when said plunger is inserted into said container.

5. An apparatus according to any preceding claim wherein said indicator is sensitive to the presence of ethylene oxide gas, temperature, and humidity for indicating sterilization efficacy of exposure to the ethylene oxide gas.

6. An apparatus according to any of Claims 1 to 4 wherein said indicator comprises an integrator which is collectively sensitive to the presence of ethylene oxide gas within said container, temperature within said container, and humidity within said container, with substantially the same sensitivity to the same conditions as the biological test spore of choice, for indicating sterilization efficacy of exposure to ethylene oxide gas.

7. An apparatus according to any preceding claim wherein said container is formed with a vent.

8. An apparatus according to any preceding claim further comprising a handle extending from said plunger.

9. An apparatus according to any preceding claim comprising means for unsealing said envelope to release said container therefrom.

10. A method for testing the efficacy of a sterilizer comprising the steps of:

placing a sterilization indicator into an apparatus comprising a container formed with an opening and having a plunger covering said opening to hold said indicator within said container;

placing the combination of said indicator and said container into an envelope;

subjecting said envelope and its contents to a sterilization cycle of ethylene oxide;

removing said container from said envelope;

retracting said plunger from said container to uncover said opening for removing said indicator therefrom; and

observing said indicator to determine the effectiveness of the sterilizer.

Fig. 1

Fig. 2

Fig. 3

Fig. 4

Fig. 5